# EUROPÄISCHE PATENTSCHRIFT

(11) **EP 0 688 210 B1**
(45) Veröffentlichungstag und Bekanntmachung des Hinweises auf die Patenterteilung: **25.02.2004**
(21) Anmeldenummer: 94911155.3
(22) Anmeldetag: 11.03.1994
(51) Int. Cl.: A61K 31/16, A61K 31/40, A61K 7/48, A61P 17/16

(54) **VERWENDUNG VON CARNOSIN IN KOMBINATION MIT UROCANINSÄURE ZUR BEHANDLUNG VON PHOTODERMATOSEN**
USE OF CARNOSINE IN COMBINATION WITH UROCANIC ACID FOR TREATMENT OF PHOTODERMATOSES
UTILISATION DE CARNOSINE EN COMBINAISON AVEC L'ACIDE UROCANIQUE POUR LE TRAITEMENT DES PHOTODERMATOSES

(30) Priorität: 13.03.1993 DE 4307983
(43) Veröffentlichungstag der Anmeldung: 27.12.1995
(73) Patentinhaber: Beiersdorf Aktiengesellschaft, 20245 Hamburg (DE)
(72) Erfinder: DEGWERT, Joachim, D-21255 Tostedt (DE); SAUERMANN, Gerhard, D-24649 Wiemersdorf (DE); SCHREINER, Volker, D-20259 Hamburg (DE); STÄB, Franz, D-21379 Echem (DE)
(86) Internationale Anmeldenummer: PCT/EP1994/000760
(87) Internationale Veröffentlichungsnummer: WO 1994/021245

(56) Entgegenhaltungen:
- EP-A- 0 413 528
- EP-A- 0 467 116
- IMMUNOLOGY Bd. 78, Nr. 1 , 1993 Seiten 99 - 104 V.E. REEVE ET AL. 'Carnosine protects from the suppression of contact hypersensitivity by ultraviolet B (280-320nm) radiation or by cis urocanic acid.'
- PATENT ABSTRACTS OF JAPAN vol. 01, no. 1222 (C-624)31. Juli 1989 & JP,A,01 117 868 (NIPPON ZOKI PHARMACEUTICAL CO LTD) 10. Mai 1989

## Beschreibung

Die vorliegende Erfindung betrifft Kosmetische und dermatologische Zubereitungen. Insbesondere betrifft die Erfindung Wirkstoffe und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenorand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus und in das Immunsystem eingreifen und Photodermatosen hervorrufen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Es wird vermutet, daß lichtinduzierte Prozesse letztendlich zu allergischen Reaktionen in der Haut führen, die sich phänotypisch als sogenannte Polymorphe Lichtdermatosen darstellen.

Um diesen Reaktionen vorzubeugen, können den kosmetischen bzw. dermatologischen Formulierungen zusätzlich Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Es ist auch bekannt, trans-Urocaninsäure (auch trans-Urocansäure, E-Urocaninsäure, E-Urocansäure, trans-(4-Imidazolyl)acrylsäure oder E-4-Imidazolylacrylsäure genannt) als UV-Filter einzusetzen. Diese Verwendung ist aber nicht Teil der vorliegenden Erfindung.

Beispiele finden sich in den japanischen Offenlegungsschriften JP-Kokai-Sho-54/027562, JP-Kokai-Sho-63/051318 und JP-Kokai-Sho-56/063965, bzw. in den dazugehörigen Auslegeschriften.

Es war indes überraschend und für den Fachmann nicht vorauszusehen, daß kosmetische und dermatologische, insbesondere topische Zubereitungen mit einem wirksamen Gehalt an Carnosin oder dessen Derivaten oder Analogen oder der Kombination der Wirkstoffe
a) Carnosin oder dessen Derivate oder Analoge und
b) Urocaninsäure oder deren Derivate oder Analoge oder jeweils Gemischen dieser Verbindungen, gegebenenfalls jeweils in Kombination mit einem oder mehreren Antioxydantien, den Nachteilen des Standes der Technik abhelfen.

Gegenstand der Erfindung ist auch die Verwendung der Kombination der Wirkstoffe
a) Carnosin oder dessen Derivate oder Analoge und
b) Urocaninsäure oder deren Derivate oder Analoge oder jeweils Gemischen dieser Verbindungen, gegebenenfalls jeweils in Kombination mit einem Antioxydans oder mehreren Antioxydantien, zur Herstellung eines Mittels zur Prophylaxe und Behandlung von lichtempfindlicher Haut, insbesondere Photodermatosen und bevorzugt der polymorphen Lichtdermatose.

Gegenstand der Erfindung ist insbesondere die Verwendung von kosmetischen und dermatologischen, insbesondere topisehen Zubereitungen mit einem wirksamen Gehalt an Carnosin oder dessen Derivaten oder Analogen in einer Kombination der Wirkstoffe enthaltend
a) Carnosin oder dessen Derivate oder Analoge und
b) Urocaninsäure oder deren Derivate oder Analoge oder jeweils Gemischen dieser Verbindungen, gegebenenfalls, jeweils in Kombination mit einem oder mehreren Antioxydantien, zur Herstellung eines Mittels zur Prophylaxe und Behandlung von lichtempfindlicher Haut, insbesondere Photodermatosen und bevorzugt der polymorphen Lichtdermatose.

Anstelle von Carnosin können auch Derivate und Analoge des Carnosins (z.B. Anserin) eingesetzt werden, insbesondere solche, aus denen sich während der Anwendung Carnosin bildet.

Anstelle von Urocaninsäure können auch Derivate und Analoge der Urocaninsäure eingesetzt werden, insbesondere solche, aus denen sich während der Anwendung Urocaninsäure bildet.

Als Carnosin oder dessen Derivate oder Analoge können bevorzugt D,L-Carnosin, L-Carnosin, D-Carnosin, D,L-Anserin, L-Anserin, D-Anserin oder deren Salze oder Säureadditionssalze, vorzugsweise wasserlösliche Salze, z.B. Natrium-, Kalium-, und Ammoniumsalze und deren Ester mit Alkoholen, z.B. Monoalkoholen oder Zuckern verwendet werden.

Bevorzugte Monoalkohole sind gesättigte oder ungesättigte, vorzugsweise einfach, zweifach oder dreifach ungesättigte Monoalkohole mit insbesondere 1 bis 18, besonders bevorzugt 14 bis 18 Kohlenstoffatomen.

Bevorzugte Ester sind Carnosinhexadecylester, Carnosin-octadecylester, Carnosinoleylester, Carnosin-Z,Z-9,12-octadecadienylester, Carnosin-Z,Z,Z-9,12,15-octadecatrienylester, Carnosin-Z,Z,Z-6,9,12-octadecatrienylester, Cholesterylcarnosin und Methyl-, Ethyl-, Butyl-, Propyl-, Amyl-, Sorbityl-, Galactosyl-, Manosyl-, Glucosyl-, Glycosyl-, und Glyceryl-Ester des Carnosins, Ester mit Thiolverbindungen, insbesondere den Thiolverbindungen, die den vorstehenden Alkoholen entsprechen.

Bevorzugt werden Carnosin oder Anserin, insbesondere L-Carnosin oder L-Anserin.

Als Urocaninsäure oder deren Derivate und Analoge können bevorzugt cis-Urocaninsäure, trans-Urocaninsäure oder deren Gemische, L-Histidin, D- und D,L-Histidin und deren Derivate oder Analoge, insbesondere physiologische Derivate und Analoge wie Imidazolglycerol, Imidazolglycerolphosphat, Imidazolacetolphosphat, 5-Amino-imidazol-4-carboxamidribonukleotid, 4-Imidazolon-5-propionsäure, Imidazolacetat, L-Histidinol, L-Histidinolphosphat, L-Histidinolacetat, L-Histidinolpalmitat, -oleat oder L-Histidinal verwendet werden.

Weiterhin bevorzugt werden Imidazol, die 2- oder 3-Pyrrolacrylsäure, 2- oder 3-Furanacrylsäure, 2-Thiophenacrylsäure, 3-Thiophenacrylsäure, wobei die Acrylsäure in der Beta-Position substituiert ist. Bevorzugt sind auch im Heterocyclus, insbesondere im Imidazolring, hydrierte Verbindungen, insbesondere Dihydrourocaninsäure.

Imidazolringe können auch in der 2-Position substituiert sein, bevorzugt werden die 2-Methyl-, 2-Ethyl-, 2-Propyl-, 2-Acetyl-Derivate, insbesondere der Urocaninsäure.

Bevorzugt werden auch die Salze und Säureadditionssalze und Ester der Urocaninsäure und deren Derivate und Analoge, insbesondere die vorstehend zu Carnosin angegebenen Salze und Ester.

Besondes bevorzugt werden Histidin, Imidazolglycerol, Imidazolglycerolphosphat, Imidazolon-5-propionsäure, Imidazolacetat, L-Histidinol, L-Histidinolphosphat, L-Histidinolacetat, L-Histidinolpalmitat, -oleat, L-Histidin, cis- oder trans-Urocaninsäure und deren Gemische.

Erfindungsgemäß kann ein Wirkstoff oder es können auch mehrere Wirkstoffe aus den Wirkstoffgruppen im Gemisch verwendet werden.

Besonders bevorzugt sind Kombinationen der Wirkstoffe von Carnosin, insbesondere L-Carnosin, und cis-Urocaninsäure oder trans-Urocaninsäure oder Gemischen von cis- und trans-Urocaninsäure oder Histidin oder Gemischen dieser Verbindungen mit zwei oder mehreren Wirkstoffen.

Weitere Bezeichnungen für die polymorphe Lichtdermatose sind PLD, PLE, Mallorca-Akne und eine Vielzahl von weite-. ren Bezeichnungen, wie sie in der Literatur (z.B. A. Voelckel et al, Zentralblatt Haut- und Geschlechtskrankheiten (1989), 156, Seite 2) angegeben sind.

Gegenstand der Erfindung sind auch die Gemische der Wirkstoffe und Zubereitungen damit.

Gegenstand der Erfindung sind ebenfalls die neuen Kombinationen der Wirkstoffe, insbesondere kosmetische und dermatologische Kombinationen.

Besonders bevorzugt werden die erfindungsgemäßen Kombinationen von L-Carnosin mit cis- und/oder trans-Urocaninsäure.

Erfindungsgemäße Zubereitungen, die zusätzlich ein UV-Schutzmittel oder Lichtschutzmittel enthalten, werden besonders bevorzugt.

Der Gegenstand der Anmeldung wird in den Ansprüchen beschrieben.

Es war nicht vorherzusehen, daß die erfindungsgemäßen Wirkstoffe oder die erfindungsgemäßen kosmetischen oder dermatolgischen Zubereitungen damit gegen PLE wirken.

Ferner war nicht vorherzusehen gewesen, daß die erfindungsgemäßen Wirkstoffe oder die erfindunsgemäßen kosmetischen oder dermatologischen Zubereitungen
- für die Anwendung genügend hohe Stabilität aufweisen
- zu hautverträglichen Produkten führen
- nicht in die hauteigene Mikroorganismenflora eingreifen
- die Hautfeuchtigkeit steigern und
- das Auswaschen der hauteigenen Urocaninsäure kompensieren würden.

Ferner war überraschend, daß die erfindungsgemäßen Wirkstoffe bei in und auf der menschlichen Haut relevanten photochemisch induzierten immunologischen Prozessen wirksam sein würden und insbesondere zur Prophylaxe und Behandlung von lichtempfindlicher Haut, insbesondere Photodermatosen und bevorzugt der polymorphen Lichtdermatose dienen können.

Zwar ist aus der DE-OS 41 21 030 bekannt, cis-Urocaninsäure in dermatologische Formulierungen zu geben, welche mancherlei Wirkung zeitigen, darunter antipsoriatische, antiallergische und dergleichen. Dieses Dokument legt . jedoch an keiner Stelle die vorteilhaften Eigenschaften der cis-Urocaninsäure auch nur nahe. Zur Prophylaxe und Behandlung der PLE führt überraschend insbesondere eine Kombination von L-Carnosin und cis- und/oder trans-Urocaninsäure und/oder Histidin zu einem Erfolg.

Die erfindungsgemäßen Wirkstoffe, Kombinationen und damit erhaltenen Zubereitungen sind prophylaktisch wirksam, indem sie die lichtempfindliche Haut schützen und die Ausbildung von PLE verhindern oder mildern. Dazu werden sie vor der Sonnenbestrahlung angewendet.

Bei manifester PLE erfolgt bei der Behandlung mit den erfindungsgemäßen Wirkstoffen und Zubereitungen eine Besserung der Hautzustande und ein schnelleres Abklingen der PLE.

Die erfindungsgemäßen kosmetischen und/oder dermatologischen Formulierungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt jeweils 0,01 Gew.-% bis 20 Gew.-%, insbesondere aber 0,1 Gew.-% bis 6 Gew.-%, bezogen auf das Gesamtgewicht des Mittels, der erfindungsgemäßen Wirkstoffe oder deren Mischungen.

Die Gewichtsanteile von Wirkstoffen der Gruppen a und b, in den Kombinationen können in einem weiten Verhältnisbereich varriert werden. Vorzugsweise beträgt das Gewichts-Verhältnis der Wirkstoffe a/b 1000:1 bis 1:1000, insbesondere 100:1 bis 1:100, besonders bevorzugt 10:1 bis 1:10.

Besonders bevorzugt werden Kombinationen mit hohem oder überwiegendem Gewichts-Anteil an Carnosin oder dessen Derivaten oder Analogen. So kann das Gewichtsverhältnis der Wirkstoffe a/b, vorzugsweise 100:1, insbesondere 10:1 betragen.

Allerdings wird auf die entsprechenden Verordnungen der einzelnen Staaten hingewiesen, welche im Einzelfalle Höchstwerte für Wirkstoffkonzentrationen festsetzen. In Deutschland ist zum gegenwärtigen Zeitpunkt die Höchstkonzentration an Urocaninsäure (cis- und trans-Isomer zusammengenommen) auf 2,0 Gew.%, bezogen auf das Gesamtgewicnt der Zusammensetzung, beschränkt.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die hervorragende Wirkung der erfindungsgemäßen Wirkstoffe und Kombinationen bei lichtempfindlicher Haut, Photodermatosen und PLD läßt sich mit in-vitro Versuchen zeigen.

So ergaben Untersuchungen an menschlicher Epidermis, die zuvor in vivo mit einer erfindungsgemäß wirksamen Kombination, z.B. aus L-Carnosin und cis-Urocaninsäure behandelt und anschließend in vitro mit UV-Licht bestrahlt worden war, daß der immunmodulierende photochemische Effekt von UV-Licht dadurch überraschenderweise aufgehoben oder zumindest gemildert werden kann. Ähnliche Ergebnisse wurden überraschenderweise erhalten, wenn man Primärkulturen von humanen Epidermiszellen in vitro bestrahlt und mit erfindungsgemäßen Kombinationen von Carnosin und Urocaninsäure cokulviert.

Diese Untersuchungen wurden mittels des sogenannten Mixed Epidermis Cell Lymphozyten Reaction Assays (MECLR) in Anlehnung an die Methodik von U.D. Cooper et al (publiziert 1985 in J. Immunol. 134, S. 129-137) durchgeführt. Humane Epidermis wurde mittels Suction Blister Methodik unter Anwendung von 200 m bar Unterdruck gewonnen (U. Kiistala et al, 1964, Lancet 1, S. 1444). Die daraus mittels Trypsinbehandlung gewonnene Zellsuspension wurde anschließend mit einer UV-Dosis von 7,5 mJ/cm² bestrahlt und in einem Verhältnis von 1:1 mit peripheren Blutleukozyten (PBL) eines allogenen Spenders in Mikrotiterplatten 6 Tage cokultiviert. Den Zellkulturansätzen wurden die erfindungsgemäßen Kombinationen an Wirksubstanzen in unterschiedlichen Konzentrationen zu Beginn der 6-tägigen Zellkultivierung hinzugefügt. Dabei erwiesen sich Kombinationen z.B. von Urocaninsäure und Carnosin in einem molaren Verhältnis von 10:1 bis 1:100 für das Verhältnis Urocaninsäure/Carnosin als besonders wirksam in der Verminderung einer UV-induzierten Immunmodulation.

Besonders bevorzugt sind solche kosmetischen und dermatologischen Zubereitungen, die in der Form eines Sonnenschutzmittels vorliegen. Vorzugsweise enthalten diese zusätzlich mindestens einen UVA-Filter und/oder mindestens einen weiteren UVB-Filter und/oder mindestens ein anorganisches Pigment.

Erfindungsgemäße kosmetische und dermatologische Zubereitungen zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasserin-Öl (W/O) oder vom Typ Öl-in-Wasser (O/W), oder eine multiple Emulsionen, beispielsweise vom Typ Wasser-in-Ölin-Wasser (W/O/W), ein Gel, einen festen Stift oder auch ein Aerosol darstellen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen
- öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme oder einer Sonnenschutzmilch sind bevorzugt und enthalten z.B. die genannten Fette, öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugte Antioxydantien sind Thiole, z.B. Cystein und Cystein-Derivate, Glutatnion, Cystin, N-Acetylcystein, Liponsäure, Folsäure, Ubiquinon, Phytinsäure, Folsäure, alpha-Hydroxysäuren z.B. Zitronensäure und Milchsäure, Zinksulfat, Zinkoxid, Vitamin C, Vitamin E, Carotin.

Die kosmetischen oder dermatologischen Zubereitungen gemäß der Erfindung enthalten Antioxidantien z.B. in Mengen von 0,01 Gew.-% bis 10 Gew.-%, vorzugsweise in Mengen von 0,5 Gew.-% bis 6 Gew.-%, insbesondere aber 2 Gew.-% bis 4 Gew.-% bezogen auf das Gesamtgewicht der Zubereitungen.

Bevorzugt können sie außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise
   3-(4-Methylbenzyliden) campher, 3-Benzylidencampher;
- 4-Aminobenzoesäure-Derivate, vorzugsweise
   4-(Dimethylamino)-benzoesäure(2-ethylhexyl)ester,
   4-(Dimethylamino)benzoesäureamylester;
- Ester der Zimtsäure, vorzugsweise
   4-Methoxyzimtsäure(2-ethylhexyl)ester,
   4-Methoxyzimtsäureisopentylester;
- Ester der Salicylsäure, vorzugsweise
   Salicylsäure(2-ethylhexyl)ester,
   Salicylsäure(4-isopropylbenzyl)ester,
   Salicylsäurehomomenthylester;
- Derivate des Benzophenons, vorzugsweise
   2-Hydroxy-4-methoxybenzophenon,
   2-Hydroxy-4-methoxy-4'-methylbenzophenon,
   2,2'-Dihydroxy-4-methoxybenzophenon;
- Ester der Benzalmalonsäure, vorzugsweise
   4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind z.B. zu nennen:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure,
   2-Methyl-5-(2-oxo-3-bornylidenmethyl)sulfonsäure und ihre Salze.

Die Liste der genannten UVB-Filter, soll selbstverständlich nicht limitierend sein.

Gegenstand der Erfindung sind auch erfindungsgemäße Zubereitungen mit Kombinationen eines UVA-Filters mit einem UVB-Filter bzw. erfindungsgemäße kosmetische oder dermatologische Zubereitungen, welche auch einen UVB-Filter enthalten.

Es kann auch von Vorteil sein, die erfindungsgemäßen Wirkstoffe mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVA-Kombination verwendeten Mengen eingesetzt werden.

Kosmetische und dermatologische Zubereitungen gemäß der Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Gegenstand der Erfindung ist auch das Verfahren zur Herstellung der erfindungsgemäßen kosmetischen Mittel, das dadurch gekennzeichnet ist, daß man in an sich bekannter Weise die erfindungsgemäßen Wirkstoffe in kosmetische und dermatologische Formulierungen einarbeitet.

Die nachfolgenden Beispiele sollen die vorliegende Erfindung verdeutlichen, ohne sie einzuschränken.

UCS bedeutet Urocaninsäure. Die Zubereitungen der Beispiele zeigen hervorragende Wirkung bei der Prophylaxe und Behandlung von empfindlicher Haut, Photodermatosen und PLD.

Die Herstellung der Zubereitungen erfolgt in an sich bekannter Weise, insbesondere durch Mischen der Bestandteile, gegebenenfalls bei erhöhter Temperatur. Fett und Wasserphasen werden getrennt hergestellt und gegebenenfalls bei erhöhter Temperatur gemischt oder emulgiert.

### Beispiel 1

| Wäßrige Zubereitung (Gesichtswasser) | |
|---|---|
| | Gew.-% |
| PEG-40-hydrogenated Castor Oil | 0,811 |
| Dipropylenglycol | 2,534 |
| PEG-8 | 1,521 |
| Na₃EDTA | 0,253 |
| Polymer JR 125 | 0,025 |
| trans-UCS | 0,750 |
| L-Carnosin | 7,000 |
| Wasser VES | ad 100,000 |
| (VES = voll entsalztes Wasser) | |

### Beispiel 2

| Wäßrige Zusammensetzung | |
|---|---|
| | Gew.-% |
| Polyfettsäureester (Cetiol HE) | 16,000 |
| PPG-3-Myristylether (Witconol APM) | 1,000 |
| Propylenglycol | 3,000 |
| Glycerin | 40,000 |
| cis-UCS | 0,500 |
| L-Carnosin | 5,000 |
| Wasser VES | ad 100,000 |

### Beispiel 3

| Hydrogel (Polyacrylatgel) | |
|---|---|
| | Gew.-% |
| Acrylsäurepolymerisat (Carbopol 934) | 1,000 |
| Tris(hydroxymethylamino)methan (Tris) | 1,000 |
| Glycerin | 2,000 |
| Propylenglycol | 2,000 |
| cis-UCS | 0,050 |
| L-Carnosin | 2,000 |
| Wasser VES | ad 100,000 |
| (statt L-Carnosin kann auch die gleiche Gewichtsmenge D,L-Carnosinpalmitylester verwendet werden) | |

### Beispiel 4

| Zubereitung mit hohem wassergehalt (sehr weich) | |
|---|---|
| | Gew.-% |
| Ceteareth (Cremopnor A 25) | 0,100 |
| Cetearyl Alcohol (Lanette 0) | 0,400 |
| Vaseline, DAB 9 | 12,500 |
| Mineralöl, DAB 9 | 11,000 |
| Ceteareth-6-stearylalkohol (Cremophor A6) | 6,000 |
| cis-UCS | 0,020 |
| L-Carnosin | 1,000 |
| Wasser VES | ad 100,000 |
| (statt cis-UCS können auch die gleichen Gewichtsmengen trans- oder cis-Dihydrourocaninsäure verwendet werden) | |

### Beispiel 5

| Zubereitung mit hohem wassergehalt (weich) | |
|---|---|
| | Gew.-% |
| Ceteareth-25 (Cremophor A25) | 1,500 |
| Cetearyl Alcohol (Lanette 0) | 8,500 |
| cis-UCS | 0,250 |
| D,L-Carnosin | 4,000 |
| Wasser VES | ad 100,000 |
| (statt cis-UCS kann auch die gleiche Gewichtsmenge trans-UCS-Galactosylester verwendet werden) | |

### Beispiel 6

| Zubereitung mit hohem Wassergehalt (weich) | |
|---|---|
| | Gew.-% |
| Ceteareth-25 (Cremophor A25) | 2,000 |
| Cetearylalcohol (Lanette 0) | 8,000 |
| Vaseline, DAB 9 | 10,000 |
| Mineralöl, DAB 9 | 10,000 |
| trans-UCS | 0,100 |
| L-Carnosin | 1,000 |
| Wasser VES | ad 100,000 |
| (statt L-Carnosin kann auch die gleiche Gewichtsmenge L-Anserin verwendet werden) | |

### Beispiel 7

| Zubereitung mit hohem Wassergehalt (mittelfest) | |
|---|---|
| | Gew.-% |
| Ceteareth-25 | 3,000 |
| Cetearyl Alcohol (Lanette O) | 17,000 |
| cis-UCS | 0,175 |
| L-Carnosin | 10,000 |
| Wasser VES | ad 100,000 |

### Beispiel 8

| Dünnflüssige Lotion | |
|---|---|
| | Gew.-% |
| Ceteareth-25 (Cremophor A25) | 1,000 |
| Ceteareth-6-stearylalcohol (Cremophor A6) | 1,000 |
| Glycerin-mono-distearat (Tegin normal) | 2,000 |
| Cetylalcohol | 1,000 |
| Isopropylmyristat | 1,450 |
| Glycerin | 1,000 |
| Polyvinylpyrrolidon | 0,500 |
| cis-UCS | 0,125 |
| L-Carnosin | 5,000 |
| Wasser VES | ad 100,000 |

### Beispiel 9

| Dickflüssige Lotion | |
|---|---|
| | Gew.-% |
| Ceteareth 25 (Cremophor A25) | 2,000 |
| Cetearyl Alcohol (Lanette 0) | 3,000 |
| Mineralöl, DAB 9 | 5,000 |
| Propylenglycol | 3,000 |
| Polyvinylpyrrolidon | 0,500 |
| cis-UCS | 0,300 |
| L-Carnosin | 2,000 |
| Wasser VES | ad 100,000 |
| (statt L-Carnosin kann auch die gleiche Gewichtsmenge L-Carnosin-Z,Z-9,12-octadecadienylester verwendet werden) | |

### Beispiel 10

| W/O-Crème | |
|---|---|
| | Gew.-% |
| Glycerinsorbitanfettsäureester | |
| (Arlacel 481) | 6,000 |
| Mikrokristallines Wachs (Lunacera M) | 1,000 |
| Neutralöl | 3,000 |
| Paraffinöl | 19,000 |
| Magnesiumstearat | 1,000 |
| Propylenglycol | 3,700 |
| Magnesiumsulfat (MgSO₄*7 H₂O) | 0,700 |
| trans-UCS | 1,000 |
| L-Carnosin | 0,800 |
| Wasser VES | ad 100,000 |
| (statt L-Carnosin kann auch die gleiche Gewichtsmenge D,L-Carnosinethylester verwendet werden) | |

### Beispiel 11

| W/O-Emulsion | |
|---|---|
| | Gew.-% |
| Polyoxyethylen-Glycerin-Sorbitan- | |
| - Fettsäureester (Arlacel 988) | 3,600 |
| Polyoxyethylen-Fettsäureester | |
| (Arlacel 989) | 1,400 |
| Cetearyl Alcohol (Lanette 0) | 2,000 |
| Mineralöl, DAB 9 | 25,000 |
| Paraben-Mischung | nach Belieben |
| Magnesiumsulfat (MgSO₄*7 H₂O) | 0,700 |
| trans-UCS | 1,250 |
| L-Carnosin | 0,600 |
| Wasser VES | ad 100,000 |

### Beispiel 12

| W/O-Lotion | |
|---|---|
| | Gew.-% |
| Glycerinsorbitanfettsäureester | |
| (Arlacel 481) | 1,300 |
| Polyoxyethylen-Fettsäureester | |
| (Arlacel 989) | 3,700 |
| Neutralöl (Miglyol) | 6,000 |
| Paraffinöl, DAB 9 | 14,000 |
| Propylenglycol | 3,800 |
| Magnesiumsulfat (MgSO₄*7 H₂O) | 0,700 |
| cis-UCS | 0,060 |
| L-Carnosin | 5,000 |
| Wasser VES | ad 100,000 |
| (statt cis-UCS kann auch die gleiche Menge cis-2-Ethylurocaninsäure verwendet werden) | |

### Beispiel 13

| O/W-Emulsion | |
|---|---|
| | Gew.-% |
| PEG-100-Stearate (Arlacel 165) | 5,000 |
| Cetearyl Alcohol (Lanette 0) | 3,000 |
| Mineralöl, DAB 9 | 25,000 |
| Paraben-Mischung | nach Belieben |
| cis-UCS | 0,325 |
| L-Carnosin | 0,500 |
| Wasser VES | ad 100,000 |
| (statt cis-UCS kann auch die gleiche Gewichtsmenge cis-UCS-Glucosylester verwendet werden) | |

### Beispiel 14

| O/W-Emulsion | |
|---|---|
| | Gew.-% |
| Polysorbate-60 (Tween 60) | 3,000 |
| Sorbitan Stearate (Arlacel 60) | 2,000 |
| Cetearyl Alcohol (Lanette 0) | 3,000 |
| Mineralöl, DAB 9 | 25,000 |
| Paraben-Mischung | nach Belieben |
| cis-UCS | 0,035 |
| L-Carnosin | 6,000 |
| Wasser VES | ad 100,000 |
| (statt cis-UCS kann auch die gleiche Gewichtsmenge cis-UCS-Sorbitylester verwendet werden) | |

### Beispiel 15

| Kationenaktive Emulsion | |
|---|---|
| | Gew.-% |
| Distearyldimethylammoniumchlorid | |
| (Genamin DS AC) | 5,000 |
| Vaseline, DAB 9 | 5,000 |
| Isopropylpalmitat | 2,000 |
| Cetylalcohol | 1,000 |
| Siliconöl | 0,100 |
| Propylparaben | 0,100 |
| Methylparaben | 0,100 |
| Glycerin | 4,000 |
| cis-/trans-UCS | 0,090 |
| L-Carnosin | 1,000 |
| Wasser VES | ad 100,000 |

### Beispiel 16

| Ionische Emulsion | |
|---|---|
| | Gew.-% |
| Natrium Cetearylsulfat (Emulgade F) | 6,000 |
| Mineralöl, DAB 9 | 25,000 |
| Paraben-Mischung | nach Belieben |
| L-Histidin | 0,450 |
| L-Carnosin | 0,250 |
| Wasser VES | ad 100,000 |
| (statt L-Carnosin kann auch die gleiche Gewichtsmenge D-Carnosin verwendet werden) | |

### Beispiel 17

| Sonnenöl | |
|---|---|
| L-Carnosin | 7,0 g |
| cis-UCS | 13,0 g |
| trans-UCS | 10,0 g |
| 3-(4'-Methylbenzyliden)campher, ("Eusolex 6300", Merck) | 60,0 g |
| Myristylalkohol, polyoxypropyliert mit 3 Mol Propylenoxid ("Witconol APM", Witco) | 608,0 g |
| C₁₂-C₁₅-Alkoholbenzoat ("Finsolv TN", Witco) Glycerinmonococoat, polyoxyethyliert mit 7 | 152,0 g |
| mol Ethylenoxid ("Cetiol HE", Henkel KGaA | 100,0 g |
| Ethanol | 65,0 g |
| 2-Octadodecanol | 20,0 g |
| Parfüm, Korrigentien, Additive, Antioxidantien, Stabilisatoren | nach Belieben |
| Die Bestandteile des Sonnenöls werden miteinander vermischt und dabei gegebenenfalls auf 40 bis 50°C zur Homogenisierung erwärmt. | |

### Beispiel 18

| Sonnengel | |
|---|---|
| L-Carnosin | 10,0 g |
| trans-UCS | 8,0 g |
| 2,4,6-Trianilino-(p-carbo-2'-ethylhexyl- 1'-oxy)-1,3,5-triazin ("Uvinul" T-150, BASF) | 25,0 g |
| Isopropylmyristat | 189,0 g |
| C₁₂-C₁₅-Alkoholbenzoat ("Finsolv TN", Witco) | 76,0 g |
| Myristylalkohol, polyoxypropyliert mit 3 Mol Propylenoxid ("Witconol APM", Witco) | 304,0 g |
| Capryl-/Caprinsäuretriglycerid ("Miglyol-Neutralöl", Dynamit-Nobel) | 195,0 g |
| "Bentone-38", Kronos-Titan | 150,0 g |
| Propylencarbonat | 20,0 g |
| Ethanol | 23,0 g |
| Parfüm, Korrigentien, Additive, Antioxidantien, Stabilisatoren | nach Belieben |

Mit den genannten Bestandteilen wird in üblicher Weise ein Sonnengel hergestellt.

### Beispiel 19

| Hydrogel | |
|---|---|
| L-Carnosin | 10,0 g |
| cis-UCS | 5,0 g |
| 2-Phenylbenzimidazol-5-sulfonsäure, ("Eusolex 232", Merck) | 27,0 g |
| Allantoin | 2,0 g |
| Sorbit fl. ("Karion F", Merck) | 22,0 g |
| "Carbopol 934", B.F. Goodrich | 15,0 g |
| Tris (hydroxymethyl)aminomethan | 27,0 g |
| Propylenglykol | 10,0 g |
| Ethanol | 300,0 g |
| Wasser | 582,0 g |
| Parfüm, Korrigentien, Additive, Antioxidantien, Stabilisatoren | nach Belieben |

Mit den genannten Bestandteilen wird in üblicher Weise ein Hydrogel hergestellt.

### Beispiel 20

| Öl-in-Wasser-Emulsion (Sonnencreme) | |
|---|---|
| L-Carnosin | 4,0 g |
| cis-UCS | 16,0 g |
| 2-Phenylbenzimidazol-5-sulfonsäure ("Eusolex 232", Merck) | 32,0 g |
| Stearylalkohol, der mit 2 Mol Ethylenoxid | |
| oxyethyliert ist ("Brij 72", ICI) | 30,0 g |
| Stearylalkohol, der mit 21 Mol Ethylenoxid oxyethyliert ist ("Brij 721", ICI) | 20,0 g |
| Cetylstearylalkohol | 25,0 g |
| Myristylalkohol, polyoxypropyliert mit 3 Mol Propylenoxid ("Witconol APM", Witco) | 64,0 g |
| C₁₂-C₁₅-Alkoholbenzoat ("Finsolv TN", Witco) | 16,0 g |
| Propylenglykol | 35,0 g |
| Tris(hydroxymethyl)aminomethan | 14,0 g |
| Wasser | 744,0 g |
| Parfüm, Korrigentien, Additive, Antioxidantien, Stabilisatoren | nach Belieben |

Die Fettkörper werden auf 80 bis 85°C erwärmt. Die wasserlöslichen Bestandteile, darunter die cis-Urocaninsäure, und l-Carnosin werden bei der gleichen Temperatur in Wasser gelöst, beide Phasen unter kräftigem Rühren miteinander vermischt, und unter mäßigerem Rühren läßt man abkühlen.

### Beispiel 21

| Öl-in-Wasser-Emulsion (Sonnencreme) | |
|---|---|
| L-Carnosin | 13,0 g |
| cis/trans-UCS (1:1) | 20,0 g |
| 2,4,6-Trianilino-(p-carbo-2'-ethylhexyl-1'-oxy)-1,3,5-triazin ("Uvinul T-150", BASF) | 18,0 g |
| C₁₂-C₁₅-Alkoholbenzoat ("Finsolv TN", Witco) | 47,0 g |
| Cetylstearylalkohol | 30,0 g |
| Gemisch aus Stearinsäuremono- und diester des Glycerins, sowie Stearinsäureester von Polyethylenoxid ("Arlacel 165", ICI) | 50,0 g |
| Myristylalkohol, polyoxypropyliert mit 3 Mol Propylenoxid ("Witconol APM", Witco) | 185,0 g |
| Wasser | 637,0 g |
| Parfüm, Korrigentien, Additive, Antioxidantien, Stabilisatoren | nach Belieben |

Die Emulsion wird entsprechend vorstehendem Beispiel zubereitet.

### Beispiel 22

| Wasser-in-Öl-Emulsion (Sonnenschutzmilch) | |
|---|---|
| L-Carnosin | 10,0 g |
| trans-UCS | 10,0 g |
| 1-(4'-tert.Butylphenyl)-3-(4'-methoxyphenyl)-propan-1,3-dion ("Parsol 1789", Givaudan) | 15,0 g |
| 4-Methoxyzimtsäure-2'-ethylhexylester, ("Parsol MCX", Givaudan) | 35,0 g |
| Ester gesättigter Fettsäuren mit Polyethylenoxid ("Arlacel 989", ICI) | 37,0 g |
| Ester ungesättigter Fettsäuren mit Glycerin und Sorbitan ("Arlacel 481", ICI) | 13,0 g |
| Myristylalkohol, polyoxypropoxyliert mit 3 Mol Propylenoxid ("Witconol APM", Witco) | 160,0 g |
| C₁₂-C₁₅-Alkoholbenzoat ("Finsolv TN", Witco) | 40,0 g |
| Magnesiumsulfat-Heptahydrat | 7,0 g |
| Wasser | 673,0 g |
| Parfüm, Korrigentien, Additive, Antioxidantien, Stabilisatoren | nach Belieben |

Die Emulsion wird in der entsprechenden Weise hergestellt wie unter Beispiel 20 beschrieben.

### Beispiel 23

| Wasser-in-Öl-Emulsion (Sonnenschutzmilch) | |
|---|---|
| L-Carnosin | 7,0 g |
| cis-UCS | 7,0 g |
| 4-Methoxyzimtsäure-2'-ethylhexylester, ("Parsol MCX", Givaudan) | 15,0 g |
| 3-(4'-Methylbenzyliden)campher ("Eusolex 6300", Merck) | 3,0 g |
| Ester ungesättigter Fettsäuren mit Glycerin und ("Arlacel 481", ICI) | 60,0 g |
| Mikrowachs ("Lunacera 11", Fuller) | 10,0 g |
| Capryl-/Caprinsäuretriglycerid ("Miglyol-Neutralöl", Dynamit-Nobel) | 20,0 g |
| Myristylalkohol, polyoxypropyliert mit 3 Mol Propylenoxid ("Witconol APM", Witco) | 145,0 g |
| C₁₂-C₁₅-Alkoholbenzoat ("Finsolv TN", Witco) | 37,0 g |
| Magnesiumstearat | 10,0 g |
| Propylenglykol | 37,0 g |
| Magnesium-Heptahydrat | 7,0 g |
| Wasser | 641,0 g |
| Parfüm, Korrigentien, Additive, Antioxidantien, Stabilisatoren | nach Belieben |

Die Emulsion wird in der entsprechenden Weise hergestellt wie unter Beispiel 20 beschrieben.

### Beispiel 24

| Wasser-in-Öl-Emulsion (Sonnenschutzmilch) | |
|---|---|
| UCS + L-carnosin (1:1) | 33,0 g |
| 4-Methoxyzimtsäure-2'-ethylhexylester ("Parsol MCX", Givaudan) | 15,0 g |
| 3-(4'-Methylbenzyliden)campher ("Eusolex 6300", Merck) | 3,0 g |
| Ester ungesättigter Fettsäuren mit Glycerin und Sorbitan ("Arlacel 481", ICI) | 60,0 g |
| Mikrowachs ("Lunacera 11", Fuller) | 10,0 g |
| Capryl-/Caprinsäuretriglycerid ("Miglyol-Neutralöl", Dynamit-Nobel) | 20,0 g |
| Myristylalkohol, polyoxypropyliert mit 3 mol Propylenoxid ("Witconol APM", Witco) | 119,0 g |
| C₁₂-C₁₅-Alkoholbenzoat ("Finsolv TN", Witco) | 30,0 g |
| Magnesiumstearat | 10,0 g |
| Propylenglykol | 37,0 g |
| Magnesiumsulfat-Heptahydrat | 7,0 g |
| Wasser | 656,0 g |
| Parfüm, Korrigentien, Additive, Antioxidantien, Stabilisatoren | nach Belieben |

Die Emulsion wird in der gleichen Weise hergestellt wie unter Beispiel 20 beschrieben.

## Patentansprüche

1. Verwendung der Kombination der Wirkstoffe
a) Camosin oder Anserin oder deren Salzen und Estern
und
b) Urocaninsäure oder Histidin oder deren Salzen und Estem oder den Analogen Imidazolglycerol, Imidazolglycerolphosphat, Imidazolacetolphosphat, 5-Aminoimidazol-4-carboxamidribonukleotid, 4-Imidazolon-5-propionsäure, Imidazolacetat, L-Histidinol, L-Histidinolphosphat, L-Histidinolacetat, L-Histidinolpalmitat oder -oleat oder L-Histidinal, Imidazol, die 2- oder 3-Pyrrolacrylsäure, 2- oder 3-Furanacrylsäure, 2-Thiophenacrylsäure, 3-Thiophenacrylsäure, im Heterocyclus, insbesondere im Imidazolring, hydrierte Verbindungen, insbesondere Dihydrourocaninsäure, wobei Verbindungen mit Imidazolringen in der 2-Position substituiert sein können, insbesondere die 2-Methyl, 2-Ethyl, 2-Propyl-2-Acetylderivate, insbesondere der Urocaninsäure, oder jeweils Gemischen dieser Verbindungen, gegebenenfalls in Kombination mit einem Antioxidans oder mehreren Antioxidantien und gegebenenfalls mit einem geeigneten Träger
zur Herstellung eines Mittels zur Prophylaxe und Behandlung von lichtempfindlicher Haut, insbesondere Photodermatosen und bevorzugt der polymorphen Lichtdermatose.

2. Verwendung nach Anspruch 1, **dadurch gekennzeichnet, daß** L-Carnosin oder L-Anserin verwendet wird.

3. Verwendung gemäß Anspruch 1, **dadurch gekennzeichnet, daß** als Wirkstoff a) Camosin, L-Camosin, Anserin oder L-Anserin und als Wirkstoff b) Histidin, Imidazolylglycerol, Imidazolylglycerolphosphat, Imidazolon-5-Propionsäure. Imidazolacetat, L-Histidinol, L-Histidinolphosphat, L-Histidinolacetat, L-Histidinolpalmitat, -oleat, L-Histidin, cis- oder trans-Urocaninsäure oder deren Gemische verwendet werden.

4. Verwendung gemäß Anspruch 1, **dadurch gekennzeichnet, daß** zusätzlich ein Lichtschutzmittel oder UV-Schutzmittel verwendet wird.

5. Verwendung nach Anspruch 1, **dadurch gekennzeichnet, daß** die kosmetischen und/oder dermatologischen Formulierungen in Form einer Lösung oder Lotion, Emulsion, eines ölig-alkoholischen oder wäßrig-atkohotischen Geles, als Stift oder als Aerosol vorliegen,

6. Verwendung nach Anspruch 1, **dadurch gekennzeichnet, daß** die kosmetischen und/oder dermatologischen Formulierungen zusätzlich als Hilfsstoffe Konservierungsmittel, Bakterizide, Parfüme, Mittel zum Verhindern des Schäumens, Farbstoffe, Pigmente, die eine färbende Wirkung haben, Verdickungsmittel, oberflächenaktive Mittel, anfeuchtende Mittel, feuchthaltende Mittel, Fette, Öle, Wachse, Alkohole, Polymole, Polymere, Schaumstabilisatoren, Elektrolyte, organische Lösungsmittel oder Siliconderivate enthalten.

7. Verwendung nach Anspruch 1, **dadurch gekennzeichnet, daß** die kosmetischen und/oder dermatologischen Formulierungen zusätzlich mindestens einen UVB-Filter und/oder mindestens einen weiteren UVA-Filter und/oder mindestens ein anorganisches Pigment enthalten.

8. Verwendung nach Anspruch 1, **dadurch gekennzeichnet, daß** der UVB-Filter ausgewählt ist aus der Gruppe bestehend aus 3-Benzylidenecampherderivaten, 4-Aminobenzoesäurederivaten, Estern der Zimtsäure, Estern der Salicylsäure, Derivaten des Benzophenons, Estern der Benzalmalonsäure, 2,4,6-Trianilino-(pcarbo-2'-ethyl-1'-hexyloxy)-1,3,5-triazin, Salzen der 2-Phenylbenzimidazole-5-sulfonsäure, Sulfonsäurederivaten von Benzophenonen und Sulfonsäurederivaten des 3-Benzylidenecamphers.

9. Verwendung nach Anspruch 1, **dadurch gekennzeichnet, daß** der UV-A-Filter ein Derivat des Dibenzoylmethans, 1-(4'-tert.Butylphenyl)-3-(4'methoxyphenyl)propan-1,3-dions oder 1-Phenyl-3-(4'-isopropylphenyl)propan-1,3-dions ist.

10. Verwendung nach Anspruch 1, **dadurch gekennzeichnet, daß** die kosmetischen oder dermatologischen Formulierungen Pigmente auf der Basis von Titandioxid enthalten.

11. Verwendung nach Anspruch 1, **dadurch gekennzeichnet, daß** die kosmetischen und/oder dermatologischen Formulierungen außerdem mindestens einen kosmetischen Hilfsstoff, ausgewählt aus oberflächenaktiven Mitteln, Verdickungsmitteln, Polymeren, Konservierungsmitteln, Schaumstabilisatoren, Elektrolyten, organischen Lösungsmitteln, Siliconderivaten, Ölen, Fetten, Wachsen, Farbstoffen, und Pigmenten enthalten.

12. Verwendung nach Anspruch 1, **dadurch gekennzeichnet, daß** zusätzlich ein Lichtschutzmittel oder UV-Schutzmittel verwendet wird.

13. Kosmetische und dermatologische, topische Zubereitungen mit einem Gehalt einer Kombination der Wirkstoffe
a) Camosin oder Anserin oder deren Salzen und Estern
und
b) Urocaninsäure oder Histidin oder deren Salzen und Estern oder den Analogen Imidazolglycerol, Imidazolglycerolphosphat, Imidazolacetolphosphat, 5-Aminoimidazol-4-carboxamidribonukleotid, 4-Imidazolon-5-propionsäure Imidazolacetat, L-Histidinol, L-Histidinolphosphat, L-Histidinolacetat, L-Histidinolpalmitat oder -oleat oder L-Histidinal, Imidazol, die 2- oder 3-Pyrrolacrylsäure, 2- oder 3-Furanacrylsäure, 2-Thiophenacrylsäure, 3-Thiophenacrylsäure, im Heterocyclus, insbesondere im Imidazolring, hydrierte Verbindungen, insbesondere Dihydrourocaninsäure, wobei Verbindungen mit Imidazolringen in der 2-Position substituiert sein können, insbesondere die 2-Methyl, 2-Ethyl, 2-Propyl-2-Acetylderivate, insbesondere der Urocaninsäure, oder jeweils Gemischen dieser Verbindungen, gegebenenfalls in Kombination mit einem Antioxidans oder mehreren Antioxidantien oder einem zusätzlichen Lichtschutzmittel oder UV-Schutzmittel.

## Claims

1. Use of the combination of the active compounds
a) carnosine or anserine or salts and esters thereof and
b) urocanic acid or histidine or salts and' esters thereof or the analogues imidazole-glycerol, imidazole-glycerol phosphate, imidazole-acetol phosphate, 5-aminoimidazole-4-carboxamide ribonucleotide, 4-imidazolone-5-propionic acid, imidazole acetate, L-histidinol, L-histidinol phosphate, L-histidinol acetate, L-histidinol palmitate or oleate or L-histidinal, imidazole, 2-or 3-pyrrole-acrylic acid, 2- or 3-furan-acrylic acid, 2-thiophene-acrylic acid, 3-thiophene-acrylic acid, compounds hydrogenated in the heterocylic radical, in particular in the imidazole ring, in particular dihydrourocanic acid, wherein compounds with imidazole rings can be substituted in the 2-position, in particular the 2-methyl, 2-ethyl, 2-propyl-2-acetyl derivatives, in particular of urocanic acid, or in each case mixtures of these compounds, optionally in combination with one antioxidant or several antioxidants and optionally with a suitable carrier
for the preparation of a composition for the prophylaxis and treatment of light-sensitive skin, in particular photodermatoses, and preferably polymorphic photodermatosis.

2. Use according to Claim 1, **characterized in that** L-carnosine or L-anserine is used.

3. Use according to Claim 1, **characterized in that** carnosine, L-carnosine, anserine or L-anserine is used as active compound a) and histidine, imidazolylglycerol, imidazolylglycerol phosphate, imidazolone-5-propionic acid, imidazole acetate, L-histidinol, L-histidinol phosphate, L-histidinol acetate, L-histidinol palmitate or oleate, L-histidine, cis- or trans-urocanic acid or mixtures thereof are used as active compound b).

4. Use according to Claim 1, **characterized in that** a light stabilizer or UV stabilizer is additionally used.

5. Use according to Claim 1, **characterized in that** the cosmetic and/or dermatological formulations are in the form of a solution or lotion, an emulsion, an oily-alcoholic or aqueous-alcoholic gel, a stick or an aerosol.

6. Use according to Claim 1, **characterized in that** the cosmetic and/or dermatological formulations additionally comprise, as auxiliaries, preservatives, bactericides, perfumes, agents for preventing foaming, dyestuffs, pigments which have a colouring action, thickeners, surface-active agents, moistening agents, humectants, fats, oils, waxes, alcohols, polymols, polymers, foam stabilizers, electrolytes, organic solvents or silicone derivatives.

7. Use according to Claim 1, **characterized in that** the cosmetic and/or dermatological formulations additionally comprise at least one UVB filter and/or at least one further UVA filter and/or at least one inorganic pigment.

8. Use according to Claim 1, **characterized in that** the UVB filter is chosen from the group consisting of 3-benzylidenecamphor derivatives, 4-aminobenzoic acid derivatives, esters of cinnamic acid, esters of salicylic acid, derivatives of benzophenone, esters of benzalmalonic acid, 2,4,6-trianilino-(p-carbo-2'-ethyl-l'-hexyloxy)-1,3,5-triazine, salts of 2-phenylbenzimidazole-5-sulphonic acid, sulphonic acid derivatives of benzophenones and sulphonic acid derivatives of 3-benzylidenecamphor.

9. Use according to Claim 1, **characterized in that** the UV-A filter is a derivative of dibenzoylmethane, 1-(4'-tert-butylphenyl)-3-(4'-methoxyphenyl)propane-1,3-dione or 1-phenyl-3-(4'-isopropylphenyl)propane-1,3-dione.

10. Use according to Claim 1, **characterized in that** the cosmetic or dermatological formulations comprise pigments based on titanium dioxide.

11. Use according to Claim 1, **characterized in that** the cosmetic and/or dermatological formulations furthermore comprise at least one cosmetic auxiliary chosen from surface-active agents, thickeners, polymers, preservatives, foam stabilizers, electrolytes, organic solvents, silicone derivatives, oils, fats, waxes, dyestuffs and pigments.

12. Use according to Claim 1, **characterized in that** a light stabilizer or UV stabilizer is additionally used.

13. Cosmetic and dermatological topical formulations with a content of a combination of the active compounds
a) carnosine or anserine or salts and esters thereof and
b) urocanic acid or histidine or salts and esters thereof or the analogues imidazole-glycerol, imidazole-glycerol phosphate, imidazole-acetol phosphate, 5-aminoimidazole-4-carboxamide ribonucleotide, 4-imidazolone-5-propionic acid, imidazole acetate, L-histidinol, L-histidinol phosphate, L-histidinol acetate, L-histidinol palmitate or oleate or L-histidinal, imidazole, 2-or 3-pyrrole-acrylic acid, 2- or 3-furan-acrylic acid, 2-thiophene-acrylic acid, 3-thiophene-acrylic acid, compounds hydrogenated in the heterocylic radical, in particular in the imidazole ring, in particular dihydrourocanic acid, wherein compounds with imidazole rings can be substituted in the 2-position, in particular the 2-methyl, 2-ethyl, 2-propyl-2-acetyl derivatives, in particular of urocanic acid, or in each case mixtures of these compounds, optionally in combination with one antioxidant or several antioxidants or an additional light stabilizer or UV stabilizer.

## Revendications

1. Utilisation de l'association des substances actives
a) carnosine ou ansérine ou leurs sels et esters
et
b) acide urocanique ou histidine ou leurs sels et esters ou les analogues imidazolglycérol, phosphate d'imidazolglycérol, phosphate d'imidazolacétol, 5-amino-imidazol-4-carboxamideribonucléotide, acide 4-imidazolone-5-propionique, acétate d'imidazole, L-histidinol, phosphate de L-histidinol, acétate de L-histidinol, palmitate de L-histidinol ou oléate de L-histidinol ou L-histidinal, imidazole, l'acide 2- ou 3-pyrrolacrylique, acide 2- ou 3-furannacrylique, acide 2-thiophénacrylique, acide 3-thiophénacrylique, des composés hydrogénés dans l'hétérocycle, en particulier dans le cycle imidazole, notamment l'acide dihydro-urocanique, les composés comportant des cycles imidazole en position 2 pouvant être substitués, en particulier les dérivés 2-méthyle, 2-éthyle, 2-propyl-2-acétyle, notamment de l'acide urocanique, ou des mélanges de ces composés, éventuellement en association avec un antioxydant ou plusieurs antioxydants et éventuellement avec un véhicule approprié,
pour la fabrication d'une composition destinée à la prophylaxie et au traitement de la peau photosensible, en particulier de photodermatoses et de préférence de la photodermatose polymorphe.

2. Utilisation selon la revendication 1, **caractérisée en ce qu'**on utilise la L-carnosine ou la L-ansérine.

3. Utilisation selon la revendication 1, **caractérisée en ce qu'**on utilise en tant que substance active a) la carnosine, la L-carnosine, l'ansérine ou la L-ansérine, et en tant que substance active b) l'histidine, l'imidazolylglycérol, le phosphate d'imidazolylglycérol, l'acide imidazolone-5-propionique, l'acétate d'imidazole, le L-histidinol, le phosphate de L-histidinol, l'acétate de L-histidinol, le palmitate de L-histidinol, l'oléate de L-histidinol, la L-histidine, l'acide *cis-* ou *trans-*urocanique ou des mélanges de ceux-ci.

4. Utilisation selon la revendication 1, **caractérisée en ce qu'**on utilise en outre un photoprotecteur ou un agent de protection contre les rayons UV.

5. Utilisation selon la revendication 1, **caractérisée en ce que** les compositions cosmétiques et/ou dermatologiques se trouvent sous forme d'une solution ou lotion, d'une émulsion, d'un gel huileux-alcoolique ou aqueux-alcoolique, sous forme d'un bâton ou d'une composition à pulvériser en aérosol.

6. Utilisation selon la revendication 1, **caractérisée en ce que** les compositions cosmétiques et/ou dermatologiques contiennent en outre en tant qu'adjuvants des conservateurs, des bactéricides, des parfums, des agents destinés à empêcher la formation de mousse, des colorants, des pigments qui ont un effet colorant, des épaississants, des agents tensioactifs, des agents hydratants, des agents retenant l'humidité, des graisses, des huiles, des cires, des alcools, des polyols, des polymères, des stabilisants de mousse, des électrolytes, des solvants organiques ou des dérivés de silicone.

7. Utilisation selon la revendication 1, **caractérisée en ce que** les compositions cosmétiques et/ou dermatologiques contiennent en outre au moins un filtre UVB et/ou au moins un autre filtre UVA et/ou au moins un pigment minéral.

8. Utilisation selon la revendication 1, **caractérisée en ce que** le filtre UVB est choisi dans l'ensemble constitué par des dérivés de 3-benzylidènecamphre, des dérivés d'acide 4-aminobenzoïque, des esters de l'acide cinnamique, des esters de l'acide salicylique, des dérivés de la benzophénone, des esters de l'acide benzalmalonique, la 2,4,6-trianilino-(p-carbo-2'-éthyl-l'-hexyloxy)-1,3,5-triazine, des sels de l'acide 2-phénylbenzimidazole-5-sulfonique, des dérivés sulfoniques de benzophénones et des dérivés sulfoniques du 3-benzylidènecamphre.

9. Utilisation selon la revendication 1, **caractérisée en ce que** le filtre UVA est un dérivé du dibenzoylméthane, de la 1-(4'-tert-butylphényl)-3-(4'-méthoxyphényl)propane-1,3-dione ou de la 1-phényl-3-(4'-iso-propylphényl)propane-1,3-dione.

10. Utilisation selon la revendication 1, **caractérisée en ce que** les compositions cosmétiques ou dermatologiques contiennent des pigments à base de dioxyde de titane.

11. Utilisation selon la revendication 1, **caractérisée en ce que** les compositions cosmétiques et/ou dermatologiques en outre contiennent au moins un adjuvant cosmétique, choisi parmi des agents tensioactifs, des épaississants, des polymères, des conservateurs, des stabilisants de mousse, des électrolytes, des solvants organiques, des dérivés de silicone, des huiles, des graisses, des cires, des colorants et des pigments.

12. Utilisation selon la revendication 1, **caractérisée en ce qu'**on utilise en outre un photoprotecteur ou un agent de protection contre les rayons UV.

13. Préparation topique cosmétique et dermatologique ayant une teneur en une association des substances actives
a) carnosine ou ansérine ou leurs sels et esters
et
b) acide urocanique ou histidine ou leurs sels et esters ou les analogues imidazolglycérol, phosphate d'imidazolglycérol, phosphate d'imidazolacétol, 5-amino-imidazol-4-carboxamideribonucléotide, acide 4-imidazolone-5-propionique, acétate d'imidazole, L-histidinol, phosphate de L-histidinol, acétate de L-histidinol, palmitate de L-histidinol ou oléate de L-histidinol ou L-histidinal, imidazole, l'acide 2- ou 3-pyrrolacrylique, acide 2- ou 3-furannacrylique, acide 2-thiophénacrylique, acide 3-thiophénacrylique, des composés hydrogénés dans l'hétérocycle, en particulier dans le cycle imidazole, notamment l'acide dihydro-urocanique, les composés comportant des cycles imidazole en position 2 pouvant être substitués, en particulier les dérivés 2-méthyle, 2-éthyle, 2-propyl-2-acétyle, notamment de l'acide urocanique, ou des mélanges de ces composés, éventuellement en association avec un antioxydant ou plusieurs antioxydants ou un photoprotecteur ou agent de protection contre les rayons UV, supplémentaire.
